# EUROPÄISCHE PATENTSCHRIFT

(11) **EP 4 376 760 B1**
(45) Veröffentlichungstag und Bekanntmachung des Hinweises auf die Patenterteilung: **25.06.2025**
(21) Anmeldenummer: 23789495.1
(22) Anmeldetag: 26.09.2023
(51) Int. Cl.: A61B 46/23, G01R 33/28, A61B 46/20, G01R 33/341, G01R 33/34

(54) **STERILTUCH VORBEREITET FÜR ODER MIT EINER MRT-LOKALSPULE SOWIE HERSTELLUNG UND VERWENDUNG EINES SOLCHEN STERILTUCHS**
STERILE DRAPE PREPARED FOR OR WITH AN MRI LOCAL COIL AND PREPARATION AND USE OF SUCH A STERILE DRAPE
CHAMP OPÉRATOIRE STÉRILE PRÉPARÉ POUR OU AVEC BOBINE LOCALE D'IRM ET PRÉPARATION ET UTILISATION D'UN TEL CHAMP STÉRILE

(30) Priorität: 28.09.2022 DE 102022125038
(43) Veröffentlichungstag der Anmeldung: 05.06.2024
(73) Patentinhaber: Noras Holding GmbH & Co KG, 97204 Höchberg (DE)
(72) Erfinder: NORAS, Manuel, 97204 Höchberg (DE); BRÜCKNER, Manuel, 97204 Höchberg (DE)
(74) Vertreter: Scharfenberger, Burkhard
(86) Internationale Anmeldenummer: PCT/DE2023/100713
(87) Internationale Veröffentlichungsnummer: WO 2024/067919

(56) Entgegenhaltungen:
- DE-U1- 202023 101 603
- US-A- 5 396 905
- US-A1- 2007 235 038
- US-A1- 2014 275 973
- US-A1- 2015 011 870
- US-A1- 2017 105 807
- US-A1- 2020 383 741

## Beschreibung

Vorliegende Erfindung betrifft ein Steriltuch für die Verwendung bei magnetresonanztomographischen Untersuchungen oder Operationen unter Einsatz magnetresonanztomographischer Bildgebung gemäß dem Oberbegriff der Ansprüche 1 bzw. 2, sowie Herstellungsverfahren und Verwendungen für derartige Steriltücher.

Bei der Durchführung von medizinischen Untersuchungen oder Operationen, bei denen es auf das genau Ansteuern oder die präzise Manipulation eines bestimmten Zielvolumen, etwa eines Entzündungsherdes, oder eines Tumors oder einer anderen auffälligen Veränderung ankommt, kann die Untersuchung bzw. Operation unter Einsatz einer magnetresonanztomographischen Bildgebung erfolgen, auf welcher Wirkung und Position der verwendeten Instrumente im bzw. relativ zum anvisierten Ziel mehr oder weniger in Echtzeit überprüft werden kann.

Dabei kommen zum Erfassen von magnetresonztomographischen Signalen aus dem Zielvolumen sogenannte Lokalspulen zum Einsatz, welche in unmittelbarer Nähe des zu Untersuchenden bzw. zu operierenden Zielbereichs gebracht werden, um eine möglichst starkes Signal aufnehmen zu können. Da die Operation bzw. Untersuchung unter sterilen Bedingungen erfolgen muss, müssen die Lokalspulen, d.h. deren Oberfläche, sterilisiert, oder die Spule steril verpackt werden. Im Stand der Technik wird letzteres bevorzugt, da dies einfacher zu bewerkstelligen ist.

Beispielsweise werden die bei der Untersuchung oder Operation einzusetzende Lokalspulen direkt vor der Operation mit (Stücken von) bekannten, zum Abdecken des Patienten verwendeten sterilen Tüchern umwickelt und mit Klebeband auf das den Patienten verdeckende Tuch geklebt. Diese immer noch häufig praktizierte Lösung ist jedoch nicht zufriedenstellend, da eine ausreichend sterile Abdeckung nicht immer garantiert werden und das Umwickeln einer üblicherweise torusförmigen Spule mit den vorhanden flächigen sterilen Tüchern schwierig ist und teilweise nur unzureichend gelingt. Zudem ist die benötigte Vorbereitungszeit sowie der Bedarf an sterilen Personal (also Personen, welche die vor einer Operation vorgeschriebene Prozedur zur möglichst weitgehenden Sterilisierung aller potentiell mit dem Patienten in Kontakt kommender Körper- und Bekleidungsoberflächen durchgeführt haben) hoch, da das Umwickeln und Festkleben der Spulen von nur einer sterilen Person alleine schwer zu bewerkstelligen ist.

Im Stand der Technik sind daher bereits ausgefeiltere Lösungen vorgeschlagen worden. Beispielsweise stellt die US-Veröffentlichungsschrift US 2015/0011870 A1 sterile Abdeckungen aus flexiblem Material zum Einpacken von Magnetresonanzlokalspulen vor, welche auf einer Innenseite mit einem Klebefilm versehen sind, sodass eine Lokalspule vergleichsweise schnell steril verpackt werden kann, indem sie auf eine Hälfte der Innenseite der Abdeckung gelegt, mit der anderen Hälfte überdeckt und die beiden Hälften sodann aneinandergepresst werden, um einen keimdichten Verschluss zu bewirken. Als Nachteil dieser Lösung verbleibt aber, dass die so steril verpackte Lokalspule noch an dem eigentlichen Einsatzort am Körper des Patienten an einen den Patienten überdeckenden großflächigen sterilen Tuch sicher und dauerhaft befestigt werden muss.

Dieser Nachteil wird zwar überwunden durch das in dem Patent US 5,396,905 gezeigte Steriltuch, jedoch wird dies mit anderen Nachteilen erkauft. Bei dem dort vorgeschlagenen Steriltuch ist die Lokalspule für die magnetresonanztomographische Untersuchung bereits werkseitig zwischen einem der bereits erwähnten bekannten sterilen Tücher zur großflächigen Abdeckung des Patienten sowie einer auf das Tuch aufgeklebten Abdeckung steril verpackt. Es wird hierbei zusätzlich vorgeschlagen die Lokalspule unterhalb dieser Abdeckung mit einem nur schwer schneidbaren Maerial zu überdecken und so vor ungewollter Beschädigung bei der Durchführung chirurgischer Operationen zu schützen. Der Nachteil des dort vorgeschlagenen Steriltuchs mit integrierter Lokalspule besteht darin, dass der Operateur auf die Verwendung einer bestimmten Spule festgelegt ist und diese nicht frei und an die Untersuchung angepasst unmittelbar vor der Durchführung des Eingriffs bzw. der Untersuchung auswählen kann, beispielsweise abhängig von der Größe oder des vorzunehmenden Einschnitts oder die Tiefe des zu erreichenden Zielpunktes.

In der Offenlegungsschrift US 2017/0105807 A1 wird ein Steriltuch vorgestellt, welches ein textiles Basispaneel, ein darauf positionierbares Zwischenpaneel und ein auf dem Zwischenpaneel positionierbares Befestigungspaneel offenbart. Die drei Paneele weisen jeweils eine Fenestration auf und werden über eine flüssigkeitsdichte Versiegelung entlang des Innenrandes der Fenestration miteinander verbunden. Das Zwischenpaneel ist flüssigsundurchdringlich und das Befestigungspaneel hat eine Oberseite aus einem Material, welches starke und flüssigkeitsfeste Klebeverbindungen eingehen kann, wie etwa ein polymerfilmüberzogenes SMS (spundbond meltblown-spundbond)-Laminat, und eine Unterseite, welche mit der Oberseite des Zwischenpaneels stark und flüssigkeitsfest verklebbar ist. Das Befestigungspaneel dient der Klebebefestigung einer Flüssigkeitssammeltasche.

Die Offenlegungsschrift US 2007/0235038 A1 schlägt Steriltücher mit Klebemöglichkeiten auf einer patientenabgewandten Oberseite zur Befestigung von chirurgischen Retraktionssystemen vor. In manchen Ausführungsformen sind die Klebemöglichkeiten als Öffnungen im Steriltuch vollständig umgebende Klebebereiche ausgeführt.

In dem Dokument US 2015/0011870 A1 sind Abdeckungen für flexible RF-Spulen offenbart, die eine klebstofffreie selbstklebende Innenseite und eine nichtklebende Außenseite haben. Zum keimdichten Abdecken wird die RF-Spule auf der Innenseite eines oberen oder unteren Tuchs platziert und ein unteres bzw. oberes Tuch mit der Innenseite nach unten aufgelegt und angedrückt, wobei sich die Innenseiten der beiden Tücher selbstklebend, aber lösbar verbinden. Eine Verbindung mit einem Steriltuch wird nicht offenbart. Vielmehr wird eine Fixierung an den Kopffixierungsdornen einer Kopfhalterung angeregt.

In der Offenlegungsschrift US 2020/383741 A1 wird ein Steriltuchsystem bestehend aus einem mit Aufbewahrungstaschen ausgestatteten Steriltuch, welches in einer besonderen Weise gefaltet und entfaltet wird, offenbart. Die Taschen beherbergen verschiedene medizinische Artikel wie bespielsweise Verbände, sterile Handschuhe, Katheter, Nadeln, Abbindschnüre oder Desinfektionsmitel.

Die Offenlegungsschrift US 2014/0275973 A1 zeigt eine schlauchartige Hülle zum keimdichten Verpacken flexibler MRT-Lokalspulen während Kopfoperationen. Die Spule wird durch eine offene Seite der Hülle eingeschoben und die Seite dann durch Falten eines überstehenden Bereichs und Verkleben mittels Klebestreifen verschlossen. Eine Verbindung mit einem Steriltuch wird nicht vorgeschlagen.

Vor diesem Hintergrund hat sich vorliegende Erfindung die Aufgabe gestellt, eine flexibler einsetzbare und leichter handhabbare Lösung für die sterile Anwendung einer MRT-Lokalspule bei einer Operation oder Untersuchung zu finden.

Gelöst wird diese Aufgabe durch ein Steriltuch gemäß Anspruch 1 oder 2, welches zur Verbindung mit einer, nicht notwendig sterilen, Lokalspule geeignet ist, ein Steriltuch-Kit gemäß Anspruch 9, sowie Verfahren zur Herstellung derartiger Steriltücher bzw. Steriltuch-Kits gemäß Anspruch 10 und einer Verwendung eines solchen Steriltuch-Kits gemäß Anspruch 13.

Vorliegende Erfindung liegt die Idee zugrunde, für die den Eingriff oder die Untersuchung durchführende Person möglichst große Freiheit zu schaffen, was Größe und Form sowie die Art der Verpackung der einzusetzenden Lokalspule angeht. Zudem wird die Vorbereitung des den Patienten überdeckende Steriltuchs, also das sterile Verpacken der Lokalspule, möglichst einfach und schnell gestaltet.

Vorliegende Offenbarung stellt in einem ersten Aspekt zwei verschiedene Varianten vor, nämlich eine erste, erfindungsgemäße, bei der ein Steriltuch bereitgestellt wird, welches für die sterile Verbindung mit einer Lokalspule vorbereitet ist, und eine zweite, nicht beanspruchte, bei der die Lokalspule bereits mit dem Steriltuch verbunden bzw. in diese integriert ist.

Wesentliches Merkmal des Steriltuchs gemäß der ersten Variante ist mindestens ein für die sterile Anbindung einer Spule vorbereiteter Bereich. Dieser ist häufig relativ zentral auf dem Tuch gelegen, kann aber auch im Randbereich des Tuches positioniert sein, sodass auch bei Positionierung an den Extremitäten des Patienten Beispielsweise am Kopf, an den Armen oder den Beinen eine großflächige, auch den Torso miterfassende Bedeckung möglich ist.

Das Steriltuch gemäß dieses ersten Aspekts besteht im Wesentlichen aus einem Tuch aus sterilem, flexiblen Material wie es für den Einsatz im OP Bereich grundsätzlich wohlbekannt ist. Dieses Tuch weist an mindestens einer Stelle einen speziell zur Verbindung mit einer Lokalspule vorbereiteten Bereich auf, der in verschiedener Weise realisiert sein kann.

In einer Ausführungsform der Offenbarung, kann der Bereich eine an ihrer Außenseite sterile Tasche aus sterilen flexiblen Material sein, beispielsweise demselben Material wie das Tuch oder aber einer transparenten Folie. In diese Tasche ist Lokalspule einleg- und steril verschließbar. Erfahrungsgemäß besteht der Bereich aus einer Klebefläche, auf der die Lokalspule selbst direkt aufgeklebt werden kann, oder sie wird in eine rigide, sterile und optional leicht sterilisierbare Verkleidung eingebettet und dann die Klebefläche aufgepresst um sie sicher zu verkleben. Im gefalteten verpackten Zustand des erfindungsgemäßen Steril Tuchs ist der ein als Klebefläche ausgestaltete Verbindungsbereich bevorzugt mit einer Schutzfolie überdeckt, um ein ungewolltes Verkleben des Steril Tuchs an sich selbst zu verhindern.

Die zweite Variante der Offenbarung ist ein Steriltuch, in welches die Lokalspule bereits fest integriert ist. Dies kann derart erfolgen, dass die Lokalspule in eine in dem Tuch befindliche Tasche eingelegt wird, und die Tasche dann anschließend verklebt oder zugenäht wird. Die Lokalspule kann auch in einer rigiden, sterilen Verkleidung eingebettet sein, welche wiederum Dauerhaft mit den Tuch verbunden ist beispielsweise durch Verkleben, Verschweißen oder Einnähen.

Als weitere mögliche Ausführungsform kann die Spule steril eingepackt sein, indem Sie mit einer flächigen Abdeckung , beispielsweise aus einem textilen Material ähnlich oder identisch dem es sterilen Tuches selbst, oder aber einer transparenten Folie, überdeckt und die Abdeckung mit dem Tuch verklebt ist, oder alternativ vernäht, verschweißt oder anderweitig dauerhaft verbunden ist.

Einer Weitere vorteilhafte Ausführungsform der Offenbarung einer bereits mit dem Steriltuch vor verbundenen Lokalspule, ist derart ausgestaltet, dass die Spule selbst direkt mit dem Tuch verklebt ist. Hierbei umfasst die Spule insbesondere bevorzugt eine leicht abwasch- bzw. sterilisierbare Kunststoffumhüllung der leitenden Elemente. Die Verklebung bzw. das Verschweißen mi dem Tuch aus sterilen und flexiblen Material des Steriltuchs erfolgt dann bevorzugt über diese Kunststoffumhüllung.

Ein zweiter Aspekt vorliegender Erfindung ist ein Herstellungsverfahren eines Steriltuchs gemäß einem der oben vorgestellten Varianten, welches gekennzeichnet ist durch die vier wesentlichen Schritte Bereitstellen eines flexiblen sterilen Tuchs, Vorbereiten des Tuchs für die Verbindung mit einer Lokalspule, Bereitstellen einer Lokalspule und Verbinden der Lokalspule mit dem Tuch. Das Vorbereiten des Tuchs für die Verbindung der Lokalspule im zweiten Schritt kann dabei entsprechend der Offenbarung erfolgen durch Aufbringen einer Tasche zum Einstecken der lokalen Spule oder erfindungsgemäß durch Aufbringen einer Klebefläche wobei die Klebefläche auch die Form eines flächigen, mit dem Tuch verschweißten oder genähten Elements aus Kunststoff, welches auf einer Seite einen Klebefilm trägt, haben kann.

Die Vorteile der Steriltücher gemäß der ersten Variante vorliegender Erfindung sind die Flexibilität was Größe und Formen der verwendeten Lokalspule angeht, welche erst kurz vor der Durchführung der Untersuchung oder Operation ausgewählt und mit dem Tuch verbunden werden können. Das Steriltuch nach beiden Varianten hat jedoch den Vorteil, vergleichsweise schnell und einfach für die Untersuchung vorbereitbar zu sein: Bei der ersten Variante wird die Vorbereitung des Steriltuchs durch Aufbringen eines zum Verbinden einer Lokalspule geeigneten Bereiches ermöglicht, so dass die unmittelbar vor der Untersuchung oder Operation erfolgende Herstellung des Tuches unter Umsetzung des Herstellungsverfahrens des zweiten Aspektes mit nur zwei Personen, von denen lediglich eine sterile Person zu sein braucht, erfolgen kann. Das Steriltuch gemäß der zweiten Variante der Offenbarung kann sogar durch lediglich eine einzelne Person schnell und effektiv verwendet werden, da es lediglich aus seiner sterilen Verpackung zu entnehmen und über Patienten zu breiten ist. Darin ist ein wesentlicher Vorteil und eine wesentliche Erleichterung gegenüber den aus dem Stand der Technik bekannten Lösungen zu sehen.

Weitere vorteilhafte Weiterbildungen sind den abhängigen Ansprüchen zu entnehmen und sollen nachfolgend im Einzelnen vorgestellt werden. Für die Fachperson ist es ersichtlich, dass vorgestellten Weiterbildungen der Offenbarung grundsätzlich, auch wenn nicht ausdrücklich erwähnt, beliebig miteinander kombiniert werden können, sofern sie sich technisch nicht gegenseitig ersichtlich ausschließen oder nicht mehr unter den Schutzumfang der Ansprüche fallen.

In manchen Ausführungsformen des Steriltuchs gemäß der ersten Variante des ersten Aspekts der Offenbarung ist der zur Verbindung mit einer Lokalspule vorbereitete Bereich des Tuches eine an einer Seite des Tuches angebrachte, zumindest an ihrer Außenseite sterile Tasche aus einem sterilen, flexiblen Material, insbesondere demselben Material wie das Tuch oder bevorzugt einer transparenten Folie.

Die Lokalspule kann in die Tasche eingelegt und die Tasche bevorzugt mit einer Lasche verschlossen werden, wobei die Lasche besonders bevorzugt an eine Unterseite eine Klebefläche aufweist, mit der sie mit dem Tuch verklebbar ist, so dass die Lokalspule vollständig steril verpackt ist.

In einer Ausführungsform des Steriltuchs gemäß des ersten Aspekts der Erfindung ist der für die Verbindung mit der Lokalspule vorbereitete Bereich einer Klebefläche zum Aufkleben entweder der Lokalspule selbst, oder einer rigiden, sterilen oder leicht sterilisierbaren Verkleidung in welche die Lokalspule eingebettet ist oder eine flächige Abdeckung aus einem flexiblen Material zum keimdichten Überdecken der Lokalspule.

In Ausführungsformen mit einer rigiden Kunststoffverkleidung zum Einbetten der Lokalspule, besteht diese Kunststoffverkleidung bevorzugt aus Polyethylenethylenketon (PEEK) oder Polypropylensulfon (PPSU).

In Ausführungsformen, in denen die Lokalspule selbst direkt mit dem Tuch verklebt wird, umfasst sie besonders bevorzugt eine Umhüllung, insbesondere eine Kunststoffumhüllung der leitenden Spulenteile.

Im Rahmen dieses Offenbarung soll unter "Umriss" nur die äußere, einen (dreidimensionalen) Gegenstand bzw. dessen Projektion auf eine Ebene einfassende geschlossene Linie sowie synonym auch für die von dieser Linie begrenzte Fläche verstanden werden. Hingegen soll "Grundriss" alle bei Projektion des Gegenstandes auf die Ebene auftretenden geschlossenen Linien bzw. die zwischen diesen Linien eingeschlossene Innenfläche bezeichnen. Umriss und Grundriss sind somit nur dann verschieden, wenn der Gegenstand Löcher oder Öffnungen aufweist, welche bei der gewählten Projektionsrichtung sichtbar sind. Beispielsweise ist somit der Umriss eines torusförmigen Gegenstandes eine Kreislinie/-scheibe (bzw. je nach Projektionsrichtung auch eine Ellipse), der Grundriss aber ein von zwei Kreislinien begrenzter Kreisring.

In Ausführungsformen, in denen eine flächige Überdeckung aus sterilen flexiblen Material zum sterilen Verpacken der Lokalspule verwendet wird, hat diese Abdeckung insbesondere bevorzugt einen Umriss, welche der Spulenklebefläche entspricht. Bei diesen Ausführungsformen kann die Abdeckung bereits werkseitig mit dem Tuch verbunden sein, insbesondere derart, dass ein Teil des Randes der Abdeckung mit einem Rand der Spulenklebefläche zusammen fällt oder sich zumindest in einem im Vergleich zu den Abmessungen der Abdeckung kleinen Abstand parallel zu dem Rand der Spulenklebefläche erstreckt. Beispielsweise können Klebefläche und Abdeckung ein rechteckigen Umriss aufweisen und die Abdeckung mit einer der Rechteckseiten mit dem Tuch verbunden sein, wobei diese Rechteckseite mit der entsprechenden Seite des Klebebereichs zusammenfällt oder zu mindestens in seiner Nähe verläuft. Die Klebefläche und oder die Abdeckung kann beispielsweise Abmessung von 10 x 10 cm, 10 x 20 cm, 15 x 15 cm , 15 x 20 cm, 15 x 25 cm, 20 x 20 cm, oder 20 x 30 cm betragen.

In anderen Ausführungsformen mit einer flächigen Abdeckung aus textilem Material handelt es sich dabei um ein zu dem Tuch hinsichtlich Form, Größe und/oder Material gleichartiges Abdecktuch, welches sich von dem Tuch dadurch unterscheiden kann, dass es keine Klebeflächen oder jedenfalls keine Spulenklebefläche, aufweist.

Tuch und Abdecktuch können weiterhin jeweils eine, insbesondere kreisrunde, Öffnung zum Durchgreifen einer steril verpackten Lokalspule aufweisen. Die Öffnungen sind dabei so angeordnet, dass, wenn Tuch und Abdecktuch gleicher Größe und Form kongruent übereinanderliegen, die Öffnungen konzentrisch sind. Die Öffnung des Abdecktuchs ist hierbei bevorzugt etwas kleiner als die des Tuches um sicherzustellen, dass die Klebefläche des Tuchs vollständig überdeckt wird, insbesondere unter Berücksichtigung dessen, dass sich das über die Lokalspule gelegte Abdecktuch beim Festkleben an der Spulenklebefläche leicht dehnen, und die Öffnung sich hierdurch etwas Vergrößern wird.

Das Tuch mit der (Spulen-)Klebefläche zur Verbindung mit der Lokalspule weist bevorzugt an einer gegenüberliegenden Seite eine weitere (Patienten-)Klebefläche zur Sicherung des Tuches an der zu untersuchenden Stelle des Patienten.

In bevorzugten Ausführungsformen der Erfindung umfasst diese ein Kit aus einem zur Verbindung mit einer Lokalspule vorbereiteten Tuch der vorbeschriebenen Art und einem Abdecktuch ohne Klebeflächen. Die beiden Tücher können im Rahmen des erfindungsgemäßen Herstellungsverfahrens werkseitig zusammen, bevorzugt aber einzeln steril verpackt und zusammen im Paket ausgeliefert werden.

In manchen Ausführungsformen mit einem Bereich in Form einer Spulenklebefläche besteht diese aus einem in das Tuch integrierten flächigen Element, insbesondere aus Kunststoff, welches insbesondere an seiner Oberseite einen Klebefilm trägt.

In manchen Ausführungsformen des Steriltuchs mit einer Spulenklebefläche entspricht deren Umriss einem vergrößerten Umriss der auf dem Tuch liegenden oder projizierten Lokalspule.

In manchen Ausführungsformen des Steriltuchs mit einer Klebefläche umfasst diese einen nicht klebenden oder zumindest an der Lokalspule, das heißt abhängig von deren Material, schlecht klebenden Spulenbereich, dessen Umriss im Wesentlichen dem der Lokalspule entspricht, so dass die Lokalspule selbst nicht oder kaum verklebt wird wenn sie auf den Klebebereich aufgelegt wird. Dieser Bereich kann besonders bevorzugt durch eine auf die Klebefläche geklebte Schutzfolie vom Grundriss der Lokalspule gebildet sein. In Ausführungsformen, in denen die Klebefläche als Ganzes von einer Schutzfolie überdeckt ist, wird diese großflächige Abdeckung der Klebefläche über den Spulenbereich, insbesondere also über die Schutzfolie vom Grundriss der Lokalspule, geklebt.

Bei den gleichen Ausführungsformen der eine Klebefläche spart diese bevorzugt einen zentralen Bereich aus, dessen Größe und Formen einen verkleinerten Umriss eine durchgehende Öffnung der Lokalspule entspricht.

In manchen Ausführungsformen des Steriltuchs gemäß der ersten Variante des ersten Aspekts der Erfindung ist der zur Verbindung mit der Lokalspule vorbereitete Bereich an einer bei bestimmungsgemäßer Verwendung des Tuches vom Patienten abgewandten Seite des Tuches angeordnet.

Bei manchen Ausführungsformen des Steriltuchs gemäß des zweiten Aspekts vorliegender Erfindung umfasst die Lokalspule bevorzugt eine Kunststoffumhüllung, welche mit dem Tuch verbunden, insbesondere verklebt oder verschweißt oder vernäht ist.

In manchen Ausführungsformen des erfindungsgemäßen Steriltuchs gemäß beider Varianten des ersten Aspektes der Erfindung oder auch eines gemäß des zweiten Aspekts der Erfindung hergestellten Steriltuchs hat dieses eine mit zu der durchgehenden Öffnung der Lokalspule konzentrische Öffnung.

In manchen Ausführungsformen des Herstellungsverfahrens eines Steriltuchs gemäß des ersten oder des zweiten Aspektes der Erfindung werden in an den zweiten Schritt anschließenden weiteren Schritten das zum Verbinden der Lokalspule vorbereitete Steriltuch gefaltet und steril verpackt.

In manchen Ausführungsformen der Herstellung eines Steriltuchs gemäß des ersten oder zweiten Aspekts der Erfindung werden die Schritte des Bereitstellens der Lokalspule und des Verbindens der Lokalspule mit dem sterilen Tuch aus flexiblen Material räumlich und zeitlich getrennt von den ersten Herstellungsschritten des Bereitstellens des sterilen, flexiblen Tuchs sowie des Vorbereitens zur Verbindung durchgeführt. Insbesondere erfolgt die Verbindung der Lokalspule erst unmittelbar vor der Verendung im Rahmen einer medizinischen Untersuchung oder Operation.

In einer besonders bevorzugten Ausführungsform der erfindungsgemäßen Herstellung werden im zweiten Schritt eine (Spulen)Klebefläche auf das Tuch aus sterilen flexiblen Material aufgebracht, welche mit einer nichtklebenden Schutzfolie überdeckt wird, das Steriltuch in einem anschließenden Schritt gefaltet und steril verpackt. Zusätzlich zur Spulenklebefläche kann auf der gegenüberliegenden Seite des Tuchs, bevorzugt konzentrisch mit der Spulenklebefläche eine Patientenklebefläche aufgebracht und ebenfalls mit einer Abdeckfolie überdeckt werden. Das Tuch kann im Bereich der Spulenklebefläche mit einer, insbesondere konzentrisch in der Spulenklebefläche angeordneten Öffnung versehen werden, deren Form derer der Öffnung einer steril zu verpackenden Lokalspule entspricht und deren Größe etwas kleiner ist als die der Öffnung der Lokalspule.

Bei manchen dieser Ausführungsformen des Herstellungsverfahrens, wird zusätzlich zu dem Tuch ein diesem hinsichtlich Material, Form und/oder Größe gleichendes Abdecktuch ohne Spulenklebefläche bereitgestellt und ebenfalls, entweder zusammen mit dem Tuch oder separat, gefaltet und steril verpackt. Tuch und Abdecktuch können an korrespondierenden Stellen mit einer Öffnung zum Durchgreifen einer nach Durchführung des vollständigen erfindungsgemäßen Herstellungsverfahrens steril zwischen Tuch und Abdecktuch eingepackten Lokalspule dient und hierfür ein der Form einer Lokalspulenöffnung entsprechende Form hat.

Die Öffnung in dem Tuch ist dabei so viel kleiner als die Spulenöffnung, dass das Abdecktuch sicher an dem in die Öffnung der konzentrisch auf die Spulenklebefläche gelegten Spule hineinragenden umlaufenden Streifen Spulenklebefläche festgeklebt werden kann. Dieser Streifen kann insbesondere eine Breite von 0,5 - 2,0 cm, insbesondere ca. 1 cm haben.

Um die beim Festkleben auftretende Dehnung auszugleichen ist die zu der Tuchöffnung korrespondierende Öffnung in dem Abdecktuch bevorzugt etwas kleiner als die Tuchöffnung, insbesondere um ca. 1 mm bis 20 mm, oder zwischen 1 und 10 %, bevorzugt 2 - 5 mm oder 2 - 5 %.

In bevorzugten Ausführungsformen des zweiten Aspektes der Erfindung umfasst im letzten Schritt der Herstellung umfasst das Verbinden der Lokalspule mit dem zum Verbinden der Spule vorbereiteten Steriltuch folgende Teilschritte: Die Verpackung des Steriltuchs wird durch eine Person, welche nicht selbst steril sein muss, geöffnet. Das Steriltuch wird sodann von einer sterilen Person aus der Verpackung herausgenommen und entfaltet. Die nicht notwendig sterile Lokalspule wird anschließend auf die Klebefläche aufgelegt, was durch eine nicht notwendig sterile Person erfolgen kann, und bevorzugt durch die gleiche Person erfolgt, welche die Verpackung geöffnet hatte. Die Lokalspule wird dabei insbesondere auf der Klebefläche in einem vorhandenen Spulenbereich, mit der mit der Spule schlecht oder nicht verklebbar ist, positioniert. In einem letzten Teilschritt erfolgt das Überdecken und keimdichte Verschließen der Spule durch auflegen einer Abdeckung und Festkleben der Abdeckung an dem sterilen, flexiblen Tuch durch vollflächiges Aufdrücken auf die Klebefläche. Dieser letzte Teilschritt muss durch eine sterile Person erfolgen, da dabei die potentiell mit dem Patienten in Kontakt kommende Außen- bzw. Oberseite der Abdeckung berührt werden muss. Bei dieser sterilen Person handelt es sich bevorzugt um dieselbe Person, die bereits im zweiten Teilschritt das Steriltuch zur Verpackung herausgenommen und entfaltet hatte. Somit kann die Vollendung der Herstellung eines erfindungsgemäßen Steriltuchs und dessen Vorbereitung für eine medizinische Untersuchung unter Einsatz magnetresonanztomographischer Bildgebung durch lediglich zwei Personen, von denen nur eine steril sein muss, schnell und einfach bewerkstelligt werden.

Weitere Vorteile, Eigenschaften und Merkmale vorliegender Offenbarung und Erfindung ergeben sich aus den nachfolgenden unter Bezugnahme auf die Figuren erläuterten Ausführungsbeispielen. Diese sollen die Erfindung lediglich erläutern und in keiner Weise einschränken.

Es zeigen:
- Figur 1:: Eine Darstellung eines zur Aufnahme einer Lokalspule vorbereiteten Steriltuchs gemäß einer ersten Ausführungsform der Offenbarung.
- Figur 2:: Eine Darstellung eines zur Verbindung mit einer Lokalspule vorbereiteten Steriltuchs gemäß einer zweiten und dritten Ausführungsform entsprechend der Erfindung.
- Figur 3:: Eine Darstellung eines zur Verbindung mit einer Lokalspule vorbereiteten Steriltuchs gemäß einer vierten Ausführungsform der Offenbarung.
- Figur 4A:: Eine Darstellung eines zur Verbindung mit einer Lokalspule vorbereiteten Steriltuchs gemäß einer fünften Ausführungsform der Erfindung.
- Figur 4B:: Eine Detaildarstellung des zur Verbindung mit einer Lokalspule vorbereiteten Bereichs des Steriltuchs aus Figur 4A.
- Figur 5A:: Eine Perspektivische Ansicht einer fünften Ausführungsform eines erfindungsgemäßen Steriltuchs umfassend ein Tuch mit einem Spulenklebebereich und ein Abdecktuch.
- Figur 5B:: Ein schematischer Schnitt durch die Ausführungsform der Figur 5A.

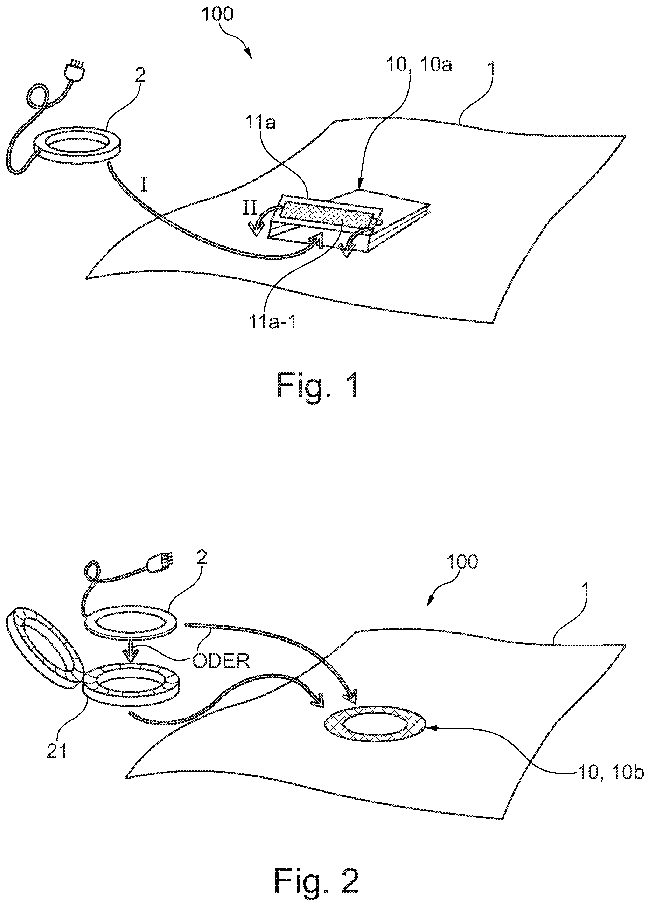

**Figur 1** zeigt eine nicht maßstabsgetreue perspektivische Ansicht eines Steriltuchs gemäß einer ersten Ausführungsform der Offenbarung. Das Steriltuch 100 umfasst ein Tuch 1 aus einem sterilen, flexiblen Material, etwa ein textiles Patientenabdecktuch, wie es im Stand der Technik wohlbekannt ist. Dieses Tuch 1 hat einen zur Verbindung mit einer Lokalspule 2 einer Magnetresonanztomographieanordnung vorbereiten Bereich 10 in Form einer Tasche 10a in welche die Lokalspule 2 einschiebbar ist, wie es der Doppelpfeil I andeutet. Die Öffnung der Tasche 10a kann nach dem vollständigen Einschieben der Spule 2 mittels der Lasche 11a keimdicht verschlossen werden, in der Figur angedeutet mittels der Doppelpfeile II. Die Lasche 11a hat hierzu an einer Unterseite einen Klebefilm 11a-1. Im Inneren der Tasche kann ein nicht dargestellter Klebefilm angebracht sein, mittels dessen der Stoff der Tasche 10a die Öffnung der Lokalspule durchgreifend auf das Tuch 1 klebbar ist.

In **Figur 2** ist eine nicht maßstabsgetreue perspektivische Ansicht zwei weitere Ausführungsformen eines erfindungsgemäßen Steriltuchs dargestellt. Mit der ersten Ausführungsform gemeinsam haben diese das flexible, sterile, insbesondere, textile, Tuch 1. Der zur Vorbereitung mit der Lokalspule auf dem Tuch 1 vorhandene Bereich 10 ist bei diesen Ausführungsformen jedoch ein kreisringförmiger Klebebereich 10b. Auf diesen kann die Lokalspule 2 gemäß einer zweiten Ausführungsform direkt aufgeklebt werden, wie es durch den oberen Doppelpfeil angedeutet ist. Alternativ kann die Lokalspule 2 in einer dritten Ausführungsform auch in eine rigide, keimdicht verschließbare, bevorzugt leicht abwasch- bzw. sterilisierbare Kunststoffverkleidung 21 eingebettet werden und diese Kunststoffverkleidung wird auf den Klebebereich 10b geklebt. Die Reihenfolge der Schritte Einbetten der Lokalspule 2 und Aufkleben der Kunststoffverkleidung 21 ist hierbei beliebig.

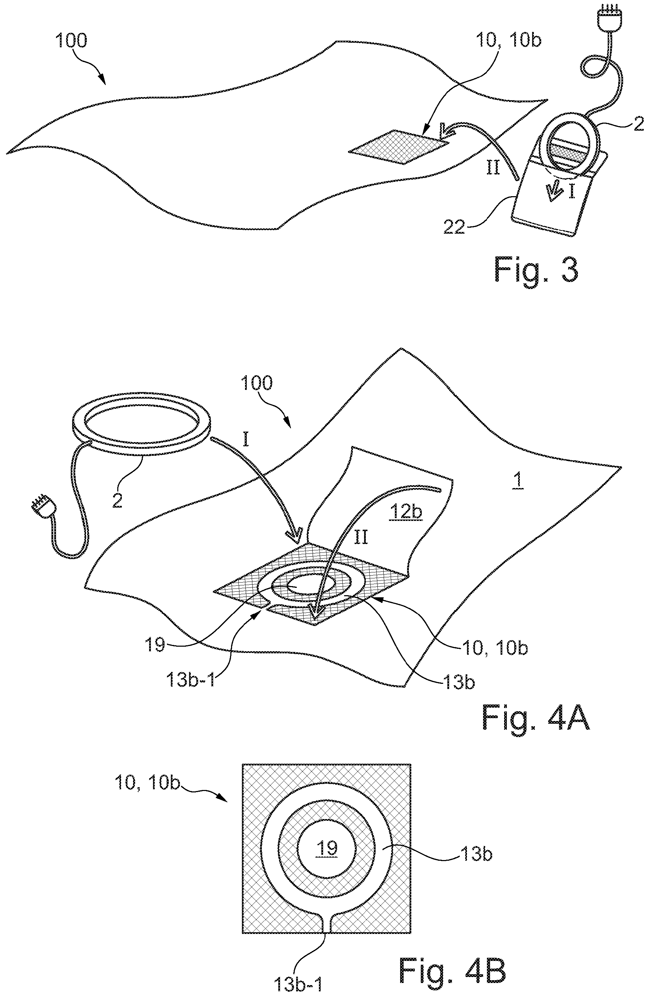

Die **Figur 3** stellt in einer weiteren nicht-maßstabsgetreuen perspektivischen Ansicht ein Steriltuch nach einer vierten Ausführungsform der Offenbarung vor. In dieser Ausführungsform trägt das bereits aus den vorbeschriebenen Ausführungsformen bekannte Tuch 1 einen rechteckigen Klebebereich 10b, auf den die in den sterilen Beutel 22 eingelegte Lokalspule 2 aufgeklebt werden kann.

Die **Figuren 4A** und **4B** zeigen verschiedene Ansichten einer fünften Ausführungsform eines erfindungsgemäßen Steriltuchs, Figur 4A eine perspektivische Ansicht des Steriltuchs als Ganzem , Figur 4B eine Draufsicht auf den Bereich 10 des Tuchs 1. Der zur Verbindung mit der Lokalspule 2 vorbereitete Bereich 10 des flexiblen, sterilen Tuchs 1 ist ein Klebebereich 10b mit rechteckigem, beispielsweise wie konkret dargestellt und besonders in Fig. 4B zu erkennen quadratischem, Umriss. In dem Klebebereich 10b ist ein kreisringförmiger Spulenbereich 13b ausgespart, auf dem die Lokalspule 2 bevorzugt positioniert wird. Hierdurch wird verhindert, dass die Spule 2 selbst am Tuch 1 verklebt wird, was das Herausnehmen der Spule nach der Untersuchung oder Operation vereinfacht und beschleunigt. An dem Tuch 1 ist eine Abdeckung 12b, deren Umriss in Form und Dimension mit dem Klebebereich 10b im Wesentlichen übereinstimmt, derart befestigt, dass ein Rand der Abdeckung 12b mit einem Rand des Klebebereiches 10b zusammenfällt. Die Abdeckung 12b ist jedoch bevorzugt aus ausreichend flexiblem Material und/oder etwas größer bemessen als der Klebebereich um die Verkürzung bei Überdeckung der, eine endliche Höhe aufweisenden Lokalspule 2 auszugleichen.

Wie durch die Doppelpfeile I und II angedeutet wird im Rahmen der erfindungsgemäßen Herstellung eines Steriltuchs mit Lokalspule aus dem zur Vorbereitung mit der Lokalspule 2 vorbereiteten Steriltuch 100 und der Lokalspule in einem ersten Schritt I die Spule auf dem Spulenbereich 13b des Klebebereichs 10b positioniert und anschließend die Abdeckung 12b über die Spule gedeckt. Anschließend wird die Abdeckung 12b flächig auf den Klebebereich 10b aufgepresst um eine vollständigen sterilen Einschluss der Lokalspule 2 sicherzustellen. Eine Kabelbereich 13b-1 des Spulenbereichs 13-b kann zur Aufnahme eines Datenkabels der Lokalspule vorhanden sein.

Das Tuch 1 gemäß dieser Ausführungsform verfügt über eine Öffnung 19. Im mit der Lokalspule 2 verbundenen Zustand ist diese Öffnung 19 mit der Öffnung der Lokalspule 2 konzentrisch.

In den vorbeschriebenen Figuren wurde ein Steriltuch gemäß der ersten Variante des ersten Aspekts der Erfindung vorgestellt, bei der die Verbindung der Lokalspule mit dem gezielt dazu vorbereiteten Steriltuch unmittelbar vor der Verwendung im Rahmen einer Untersuchung oder Operation erfolgt. Grundsätzlich können alle vorbeschriebenen Ausführungsformen auch gemäß der zweiten Variante des ersten Aspekts der Offenbarung bereits werkseitig mit einer steril verpackten Lokalspule versehen sein.

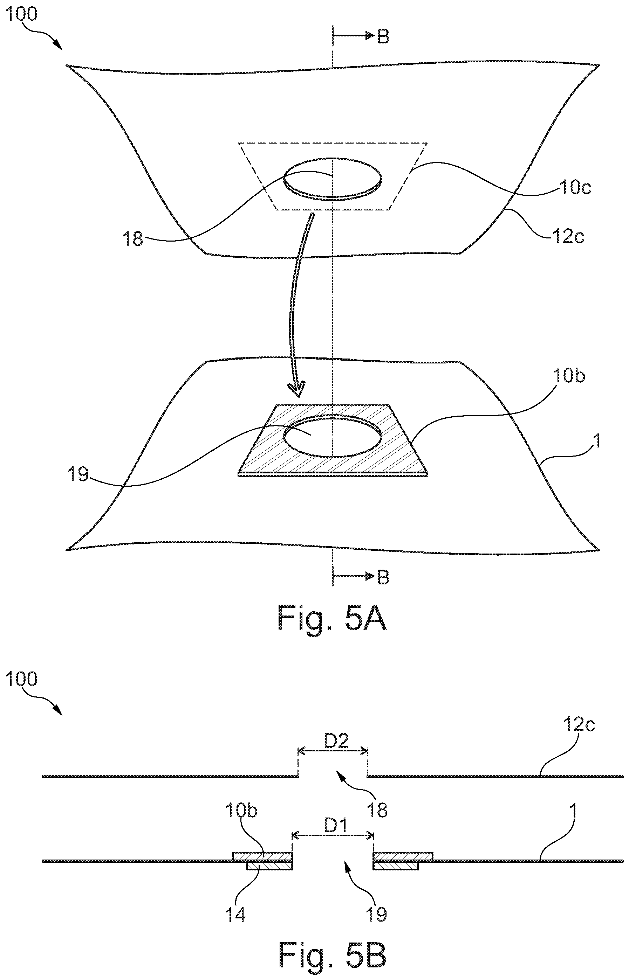

Die Figuren 5A und 5B zeigen zwei Ansichten einer weiteren bevorzugten Ausführungsform der Erfindung.

Das Steriltuch 100 dieser Ausführungsform umfasst ein Tuch 1 mit einem Spulenklebebereich 10b zum auf einer Oberseite und einer darin mittig angeordneten kreisrunden Öffnung 19. Wie in dem schematischen Schnitt der Fig. 5B zu erkennen ist auf der dem Spulenklebebereich gegenüberliegenden Unterseite ein weiterer die Öffnung 19 umgebender Patientenklebebereich 14 vorgesehen, der dazu dient, das Steriltuch 100 an der zu Untersuchenden Stelle des Patienten zu fixieren. Spulenklebebereich 10b und Patientenklebebereich 14 können, wie dargestellt, unterschiedlich groß sein. Alternativ sind sie auch gleich groß und im Übrigen auch gleichartig gestaltet, so dass sie beide jeweils als Spulen- und Patientenklebebereich dienen können. Dies hat den Vorteil, dass Anwender, wenn sie das Tuch 1 über den Patienten breiten, nicht auf die Orientierung des Tuches 1 achten müssen.

Zum Abdecken und sterilen Verpacken einer (nicht dargestellten) Lokalspule umfasst die vorliegende Ausführungsform des Steriltuchs 100 das Abdecktuch 12c, welches, wie dargestellt in seiner Größe und Form, aber auch hinsichtlich des (textilen) Materials gleich dem Tuch 1 gewählt sein kann. Das Abdecktuch 1 hat an einer zur Position der Öffnung 19 im Tuch 1 korrespondierenden Position eine ebenfalls kreisrunde Öffnung 18, welche aber nicht von einem Klebebereich umgeben sein muss. Der Durchmesser D2 der Öffnung 18 des Abdecktuchs 12c ist bevorzugt etwas kleiner gewählt als der Durchmesser D1 der Öffnung 19 des Tuchs 1, wie dies aus Fig. 5B ersichtlich ist.

Zum sterilen Einpacken der Lokalspule, bei der Durchführung des erfindungsgemäßen Herstellungsverfahrens in der Form, dass die Verbindung mit der Lokalspule räumlich und/oder zeitlich getrennt von der Bereitstellung und Vorbereitung des Tuchs 1 erfolgt, wird diese von einer nicht notwendig sterilen Person auf dem Spulenklebebereich des Tuchs 1 positioniert und zwar vorteilhafterweise derart, dass eine Öffnung der Lokalspule konzentrisch zu der Öffnung 19 zu liegen kommt. Anschließend wird, wie durch den Doppelpfeil in Figur 5A angedeutet, das Abdecktuch 12c derart auf das Tuch 1 gelegt, dass die Öffnung 18 konzentrisch zur Öffnung 19 ist, so dass der in Figur 5A gestrichelt gezeigte, die Öffnung 18 umgebende Bereich 10c auf dem Spulenklebebereich zu liegen kommt und an diesem zumindest teilweise (dort wo dies nicht durch die dazwischenliegende Lokalspule verhindert wird) festgeklebt wird und zwar durch eine sterile Person, um die äußere Sterilität des Steriltuchs 100 nicht zu gefährden. Weiterhin bevorzugt kann auch darauf geachtet werden, dass die Ecken des Abdecktuchs 12c auf denen des Tuchs 1 zu liegen kommen. Dies ist jedoch nicht unbedingt erforderlich.

Durch den im Vergleich zum Durchmesser D1 der Tuchöffnung 19 kleineren Durchmessers D2 der Abdecktuchöffnung 18 wird gewährleistet, dass trotz der beim Festkleben des Bereichs 10c des Abdecktuchs 12c am Spulenklebebereich 10b des Tuchs 1 aufgrund der Dicke der Lokalspule auftretenden Dehnung der Öffnung 18 kein Teil des Spulenklebebereichs 10b freiliegt.

### Bezugszeichenliste

- 100: Steriltuch
- 1: Tuch
- 10: vorbereiteter Bereich
- 10a: Tasche
- 11a: Lasche
- 11a-1: Klebefilm
- 10b: Spulenklebebereich
- 10c: Bereich von 12c, der auf 10b zu liegen kommt
- 12b: Abdeckung
- 12c: Abdecktuch
- 13b: Spulenbereich
- 13b-1: Kabelbereich
- 14: Patientenklebebereich
- 18: Öffnung in 12c
- 19: Öffnung in 1

- D1: Durchmesser von 19
- D2: Durchmesser von 18

- 2: Lokalspule
- 21: rigide Kunststoffverkleidung
- 22: flexibler steriler Beutel

## Patentansprüche

1. Steriltuch (100) zum Abdecken von Patienten bei magnetresonanztomographischen Untersuchungen oder Operationen unter Einsatz magnetresonanztomographischer Bildgebung, umfassend ein Tuch (1) aus sterilem, flexiblem Material, welches einen Bereich (10) aufweist, in dem es für die Verbindung mit einer Lokalspule (2) einer Magnetresonanzanordnung vorbereitet ist,
wobei der Bereich (10) eine Spulenklebefläche (10b) zum Aufkleben der Lokalspule (2) selbst, einer rigiden, sterilen oder leicht sterilisierbaren Verkleidung°(21), insbesondere aus Kunststoff, in welche die Lokalspule (2) eingebettet ist, oder einer flächigen Abdeckung (12b, 12c) zum keimdichten Überdecken der Lokalspule ist,
**dadurch gekennzeichnet, dass**
das Steriltuch (100) weiterhin eine rigide, sterile oder leicht sterilisierbare Kunststoffverkleidung (21) umfasst, welche auf der Spulenklebefläche (10b) aufklebbar oder aufgeklebt ist und in welche die Lokalspule einbettbar ist.

2. Steriltuch (100) zum Abdecken von Patienten bei magnetresonanztomographischen Untersuchungen oder Operationen unter Einsatz magnetresonanztomographischer Bildgebung, umfassend ein Tuch (1) aus sterilem, flexiblem Material, welches einen Bereich (10) aufweist, in dem es für die Verbindung mit einer Lokalspule (2) einer Magnetresonanzanordnung vorbereitet ist,
wobei der Bereich (10) eine Spulenklebefläche (10b) zum Aufkleben der Lokalspule (2) selbst, einer rigiden, sterilen oder leicht sterilisierbaren Verkleidung°(21), insbesondere aus Kunststoff, in welche die Lokalspule (2) eingebettet ist, oder einer flächigen Abdeckung (12b, 12c) zum keimdichten Überdecken der Lokalspule ist,
**dadurch gekennzeichnet, dass**
das Steriltuch (100) weiterhin eine flächige Spulenabdeckung (12b, 12c) aus einem sterilen, flexiblen Material zum sterilen Überdecken der Lokalspule (2) umfasst, wobei die Spulenabdeckung (12b, 12c) aus einem sterilen, flexiblen Material, insbesondere demselben Material wie das Tuch (1) oder einer, bevorzugt transparenten, Folie, besteht, und wobei die Spulenabdeckung (12b, 12c)
- einen Umriss aufweist, welcher dem Umriss der Spulenklebefläche (10b) entspricht, und mit dem Tuch (1) derart verbunden ist, dass ein Teil eines Randes der Spulenabdeckung (12b) mit einem Rand der Spulenklebefläche (10b) zusammenfällt oder sich zumindest in einem im Vergleich zu den Abmessungen der Spulenabdeckung (12b) kleinen Abstand parallel zu dem Rand der Spulenklebefläche (10b) erstreckt,
oder
- ein separates Abdecktuch (12c) ist, welches in Größe, Material und Form dem Tuch (1) entspricht, jedoch keine Spulenklebefläche aufweist.

3. Steriltuch nach Anspruch 2, wobei das Tuch (1) ein innerhalb der Spulenklebefläche (10b) angeordnete, insbesondere kreisförmige, Tuchöffnung und die Spulenabdeckung (12b, 12c) eine gleichartig geformte Abdeckungsöffnung aufweist, und die Abdeckung derart auf der Klebefläche (10b) anordenbar ist, dass die Abdeckungsöffnung konzentrisch zu der Tuchöffnung liegt.

4. Steriltuch gemäß dem vorhergehenden Anspruch, wobei ein Durchmesser der Abdeckungsöffnung kleiner ist als ein Durchmesser der Tuchöffnung, insbesondere um 0,5 bis 2 cm oder 1 % bis 10 % kleiner.

5. Steriltuch nach einem der vorhergehenden Ansprüche, wobei die Spulenklebefläche (10b) ein in das Tuch (1) integriertes flächiges Element aus rigidem Kunststoff (15b) ist.

6. Steriltuch nach einem der vorhergehenden Ansprüche, wobei die Spulenklebefläche (10b) einem vergrößerten Umriss der auf dem Tuch (1) liegenden oder projizierten Lokalspule (2) entspricht.

7. Steriltruch nach einem der vorhergehenden Ansprüche, wobei die Spulenklebefläche (10b) einen nichtklebenden, oder zumindest an der Lokalspule (2) schlecht klebenden Spulenbereich (13b) aufweist, dessen Umriss dem der Lokalspule (2) entspricht, so dass die Lokalspule selbst nicht oder nur leicht lösbar verklebt wird, wobei der Spulenbereich (13b) insbesondere aus einer auf eine durchgehende Klebefläche (10b) geklebte Schutzfolie vom Grundriss der Lokalspule (2) gebildet ist.

8. Steriltuch nach einem der vorhergehenden Ansprüche, wobei es auf der der Spulenklebefläche (10b) gegenüberliegenden Seite des Tuchs (1) eine Patientenklebefläche (14) aufweist, die insbesondere konzentrisch oder in Deckung zur Spulenklebefläche (10b) angeordnet ist.

9. Steriltuch-Kit umfassend ein Steriltuch gemäß Anspruch 2 in der Alternative mit einem separaten Abdecktuch (12c), wobei die Spulenklebefläche (10b) und eine ggf. vorhandene Patientenklebefläche (14) mit einer nichtklebenden Folie abgeckt ist und das Tuch (1) und das Abdecktuch (12c) jeweils einzeln steril verpackt sind.

10. Herstellung eines Steriltuchs oder eines Steriltuch-Kits nach einem der vorhergehenden Ansprüche in Vorbereitung einer magnetresonsanztomographischen Untersuchung oder Operation eines bzw. an einem Patienten, **gekennzeichnet durch** die Schritte
a. Bereitstellen eines flexiblen, sterilen Tuchs (1) und einer flächigen Spulenabdeckung (12b, 12c) oder einer rigiden Verkleidung (21) zum keimdichten Verdecken bzw. Einbetten einer Lokalspule (2),
b. Vorbereiten des Tuchs (1) für die Verbindung mit einer Lokalspule (2) und/oder der Spulenabdeckung (12b, 12c) bzw. der Verkleidung (21) durch Aufbringen einer Klebefläche (10b), welche insbesondere mit einer nichtklebenden Folie abgedeckt wird.
c. Bereitstellen einer Lokalspule (1),
d. Verbinden der Verkleidung (21) oder der Lokalspule (2) und/oder der Spulenabdeckung (12b, 12c) mit dem Tuch (1).

11. Herstellung nach dem vorhergehenden Anspruch, wobei die Schritte c und d von den vorhergehenden Schritten räumlich und/oder zeitlich getrennt, insbesondere die vorhergehenden Schritte werksseitig, die Schritte c und d hingegen erst an einem Einsatzort des Steriltuchs durchgeführt werden.

12. Herstellung nach dem vorhergehenden Anspruch, wobei die Klebefläche in Schritt b mit einer nichtklebenden Folie bedeckt wird, welche vor oder zu Beginn von Schritt d entfernt wird, und weiterhin in Schritt a als Spulenabdeckung (12b, 12c) eine Abdecktuch (12c) bereitgestellt wird und in Schritt b Tuch (1) und Abdecktuch (12c) einzeln steril verpackt werden, sodass nach Schritt b ein Steriltuch-Kit gemäß Anspruch 9 vorliegt.

13. Verwendung eines Steriltuchs gemäß einem der Ansprüche 1 - 8 oder eines Steriltuch-Kits nach Anspruch 9 umfassend die von einer sterilen ersten und einer nicht notwendig sterilen zweiten Person nicht notwendig in der nachfolgend wiedergegebenen Reihenfolge durchgeführten Teilschritte:
i. Öffnen der Verpackung des Steriltuchs durch die zweite Person, wobei diese dabei das Steriltuch (100) selbst nicht berührt,
ii. Herausnehmen und Entfalten des Steriltuchs (100) sowie abziehen der Schutzfolie durch die erste Person,
iii. Ablegen des entfalteten Steriltuchs (100),
iv. In-Kontakt-Bringen der nicht notwendig sterilen Lokalspule (2) mit der Spulenklebefläche (10b) durch die zweite Person, insbesondere derart, dass die Lokalspule (2) dabei auf einem nicht- oder schlecht klebenden Spulenbereich (13b) positioniert wird,
v. Festkleben der Abdeckung an dem Steriltuch (100) durch vollflächiges Aufdrücken durch die erste Person auf den Klebebereich (10b), sodass die Lokalspule (2) vollständig steril eingeschlossen wird.

14. Verwendung nach dem vorhergehenden Anspruch unter Verwendung eines Steriltuch-Kits nach Anspruch 9, wobei entweder
- in Teilschritt ii die nichtklebende Folie auch der Patientenklebefläche (14) entfernt und in Teilschritt iii das entfaltete Steriltuch mit der Spulenklebefläche nach oben derart auf dem Patienten abgelegt wird, dass sich die Spulenklebefläche (10b) und insbesondere eine darin enthaltene Öffnung an einer zu untersuchenden oder operierenden Stelle liegen und in Teilschritt v die Spule (2) mit dem Abdecktuch (12c) überdeckt wird,
oder
- zunächst das Abdecktuch (12c) mit der nicht mit der Spule in Kontakt kommenden Seite zuunterst auf eine sterile Unterlage gelegt, anschließend die Lokalspule (2), insbesondere mit ihrer Öffnung konzentrisch zu einer Öffnung des Abdecktuchs (12c) auf diese gelegt und dann gemäß der Teilschritte iii und iv das Steriltuch mit der Spulenklebefläche über der Lokalspule und insbesondere mit einer Öffnung des Tuchs (1) konzentrisch zur Lokalspulenöffnung, auf das Abdecktuch (12c) gelegt und anschließend Teilschritt v ausgeführt wird.

## Claims

1. A sterile drape (100) for covering patients during magnetic resonance imaging examinations or surgeries using magnetic resonance imaging, comprising a cloth (1) made of sterile, flexible material, which has a region (10) in which it is prepared for connection to a local coil (2) of a magnetic resonance system,
wherein the region (10) is a coil adhesive surface (10b) for adhering the local coil (2) itself, a rigid, sterile, or easily sterilizable covering (21), in particular made of plastic, in which the local coil (2) is embedded, or a flat cover (12b, 12c) for covering the local coil in a germ-tight manner,
**characterized in that**
the sterile drape (100) further comprises a rigid, sterile, or easily sterilizable plastic covering (21) which is or can be glued to the coil adhesive surface (10b) and in which the local coil can be embedded.

2. A sterile drape (100) for covering patients during magnetic resonance imaging examinations or surgeries using magnetic resonance imaging, comprising a cloth (1) made of sterile, flexible material, which has a region (10) in which it is prepared for connection to a local coil (2) of a magnetic resonance system,
wherein the region (10) is a coil adhesive surface (10b) for adhering the local coil (2) itself, a rigid, sterile, or easily sterilizable covering (21), in particular made of plastic, in which the local coil (2) is embedded, or a flat cover (12b, 12c) for covering the local coil in a germ-tight manner,
**characterized in that**
the sterile drape (100) further comprises a flat coil cover (12b, 12c) made of a sterile, flexible material for sterilely covering the local coil (2) wherein the coil cover (12b, 12c) consists of a sterile, flexible material, in particular the same material as the cloth (1) or a preferably transparent film, and
wherein the coil cover (12b, 12c)
- has an outline corresponding to the outline of the coil adhesive surface (10b) and is connected to the cloth (1) such that a portion of an edge of the coil cover (12b) coincides with an edge of the coil adhesive surface (10b) or extends parallel to the edge of the coil adhesive surface (10b) at least at a small distance compared to the dimensions of the coil cover (12b),
or
- is a separate covering cloth (12c) which corresponds in size, material, and shape to the cloth (1), but does not have a coil adhesive surface.

3. The sterile drape according to claim 2, wherein the cloth (1) has a, particularly circular, cloth opening arranged within the coil adhesive surface (10b) and the coil cover (12b, 12c) has a similarly shaped cover opening, and the cover can be arranged on the adhesive surface (10b) in such a way that the cover opening is concentric with the cloth opening.

4. The sterile drape according to the preceding claim, wherein a diameter of the cover opening is smaller than a diameter of the cloth opening, in particular by 0.5 to 2 cm or 1% to 10% smaller.

5. The sterile drape according to one of the preceding claims, wherein the coil adhesive surface (10b) is a flat element made of rigid plastic (15b) integrated into the cloth (1).

6. The sterile drape according to one of the preceding claims, wherein the coil adhesive surface (10b) corresponds to an enlarged outline of the local coil (2) lying on or projected onto the cloth (1).

7. The sterile drape according to one of the preceding claims, wherein the coil adhesive surface (10b) has a non-adhesive, or at least poorly adhesive, coil region (13b) whose outline corresponds to that of the local coil (2), so that the local coil itself is not glued or is easily releasable,
wherein the coil region (13b) is formed in particular from a protective film of the outline of the local coil (2) glued to a continuous adhesive surface (10b).

8. The sterile drape according to one of the preceding claims, wherein it has a patient adhesive surface (14) on the side of the cloth (1) opposite the coil adhesive surface (10b), which is arranged in particular concentrically or in alignment with the coil adhesive surface (10b).

9. A sterile cloth kit comprising a sterile cloth according to claim 2 in the alternative with a separate covering cloth (12c), wherein the coil adhesive surface (10b) and any patient adhesive surface (14) are covered with a non-adhesive film and the cloth (1) and the covering cloth (12c) are each individually sterilely packaged.

10. Production of a sterile drape or a sterile drape kit according to one of the preceding claims in preparation for a magnetic resonance imaging examination or surgery of or on a patient, **characterized by** the steps:
a. providing a flexible, sterile cloth (1) and a flat coil cover (12b, 12c) or a rigid covering (21) for germ-tight covering or embedding of a local coil (2),
b. preparing the cloth (1) for connection to a local coil (2) and/or the coil cover (12b, 12c) or the covering (21) by applying an adhesive surface (10b), which is covered in particular with a non-adhesive film,
c. providing a local coil (2),
d. connecting the covering (21) or the local coil (2) and/or the coil cover (12b, 12c) to the cloth (1).

11. Production according to the preceding claim, wherein steps c and d are spatially and/or temporally separated from the preceding steps, in particular the preceding steps are carried out at the factory, whereas steps c and d are only carried out at a place of use of the sterile drape.

12. Production according to the preceding claim, wherein the adhesive surface is covered in step b with a non-adhesive film, which is removed before or at the beginning of step d, and further in step a a covering cloth (12c) is provided as a coil cover (12b, 12c) and in step b the cloth (1) and the covering cloth (12c) are individually sterilely packaged, so that after step b a sterile drape kit according to claim 9 is present.

13. Use of a sterile drape according to any one of claims 1-8 or of a sterile drape kit according to claim 9, comprising the substeps performed by a sterile first person and a not necessarily sterile second person not necessarily in the following order:
i. Opening the packaging of the sterile drape by the second person, whereby the second person does not touch the sterile drape (100) itself,
ii. Removing and unfolding the sterile drape (100) and peeling off the protective film by the first person,
iii. Putting down the unfolded sterile drape (100),
iv. Bringing the not necessarily sterile local coil (2) into contact with the coil adhesive surface (10b) by the second person, in particular such that the local coil (2) is positioned on a non-adhesive or poorly adhesive coil area (13b),
v. Adhering the cover to the sterile drape (100) by pressing it fully onto the adhesive area (10b) by the first person, so that the local coil (2) is completely enclosed in a sterile manner.

14. Use according to the preceding claim using a sterile drape kit according to claim 9, wherein either
- in sub-step ii, the non-adhesive film is also removed from the patient adhesive surface (14), and in sub-step iii, the unfolded sterile drape is placed on the patient with the coil adhesive surface facing upwards such that the coil adhesive surface (10b) and in particular an opening contained therein are located at a site to be examined or operated on, and in sub-step v, the coil (2) is covered with the cover cloth (12c),
or
- first, the covering cloth (12c) is placed on a sterile surface with the side which is not to be brought into contact with the coil at the bottom, then the local coil (2), in particular with its opening concentric with an opening in the covering cloth (12c), is placed thereon, and then, according to sub-steps iii and iv, the sterile drape is placed with the coil adhesive surface over the local coil and in particular with an opening in the cloth (1) concentric to the local coil opening, placed on the covering cloth (12c) and then sub-step v is carried out.

## Revendications

1. Champ operatoire stérile (100) destiné à couvrir les patients lors d'examens ou d'interventions chirurgicales par imagerie par résonance magnétique, comprenant un tissu (1) en matériau stérile et flexible, doté d'une zone (10) destinée à être connectée à une bobine locale (2) d'un système de résonance magnétique,
dans lequel la zone (10) est constituée d'une surface adhésive (10b) destinée à coller la bobine locale (2), d'un revêtement rigide, stérile ou facilement stérilisable (21), notamment en plastique, dans lequel la bobine locale (2) est intégrée, ou d'un revêtement plat (12b, 12c) destiné à recouvrir la bobine locale de manière étanche aux germes,
**caractérisé en ce que**
le champ stérile (100) comprend en outre un revêtement plastique rigide, stérile ou facilement stérilisable (21) collé ou pouvant être collé à la surface adhésive (10b) et dans lequel la bobine locale peut être insérée.

2. Champ operatoire stérile (100) destiné à couvrir les patients lors d'examens ou d'interventions chirurgicales par imagerie par résonance magnétique comprenant un tissu (1) en matériau stérile et flexible, doté d'une zone (10) destinée à être connectée à une bobine locale (2) d'un système de résonance magnétique,
dans lequel la zone (10) est constituée d'une surface adhésive (10b) destinée à coller la bobine locale (2), d'un revêtement rigide, stérile ou facilement stérilisable (21), notamment en plastique, dans lequel la bobine locale (2) est intégrée, ou d'un revêtement plat (12b, 12c) destiné à recouvrir la bobine locale de manière étanche aux germes,
**caractérisé en ce que**
le champ stérile (100) comprend en outre un revêtement de bobine (12b, 12c) plate en matériau stérile et flexible destinée à recouvrir la bobine locale (2) de manière stérile, dans lequel le revêtement de bobine (12b, 12c) est constitué d'un matériau stérile et flexible, notamment du même matériau que le tissu (1) ou, de préférence, d'un film transparent, et dans lequel le revêtement de bobine (12b, 12c)
- présente un contour correspondant à celui de la surface adhésive de la bobine (10b) et est relié au tissu (1) de telle sorte qu'une partie d'un bord du revêtement de bobine (12b) coïncide avec un bord de la surface adhésive de la bobine (10b) ou s'étend parallèlement à ce bord, au moins à une faible distance par rapport aux dimensions du revêtement de bobine (12b),
ou
- est un tissu de recouvrement séparé (12c) dont la taille, le matériau et la forme correspondent au tissu (1), mais qui n'a pas de surface adhésive.

3. Champ operatoire stérile selon la revendication 2, dans lequel le tissu (1) présente une ouverture, particulièrement circulaire, ménagée dans la surface adhésive (10b) de la bobine, et le revêtement de bobine (12b, 12c) présente une ouverture de forme similaire, et le revêtement de bobine peut être disposé sur la surface adhésive (10b) de manière à ce que l'ouverture du revêtement soit concentrique à l'ouverture du tissu.

4. Champ operatoire stérile selon la revendication précédente, dans lequel un diamètre de l'ouverture du revêtement est inférieur a un diamètre de l'ouverture du tissu, notamment de 0,5 à 2 cm ou de 1 à 10 %.

5. Champ operatoire stérile selon l'une des revendications précédentes, dans lequel la surface adhésive (10b) de la bobine est un élément plat en plastique rigide (15b) intégré au tissu (1).

6. Champ operatoire stérile selon l'une des revendications précédentes, dans lequel la surface adhésive de la bobine (10b) correspond à un contour agrandi de la bobine locale (2) posée ou projetée sur le tissu (1).

7. Champ operatoire stérile selon l'une des revendications précédentes, dans lequel la surface adhésive (10b) de la bobine présente une zone (13b) non adhésive, ou du moins peu adhésive, dont le contour correspond à celui de la bobine locale (2), de telle sorte que la bobine locale elle-même n'est pas collée ou est facilement détachable, la zone (13b) etant notamment formée d'un film protecteur du contour de la bobine locale (2) collé sur une surface adhésive continue (10b).

8. Champ operatoire stérile selon l'une des revendications précédentes, **caractérisé en ce qu'**il présente une surface adhésive (14) destinée au patient sur le côté du tissu (1) opposé à la surface adhésive (10b) de la bobine, qui est disposée notamment de manière concentrique ou alignée avec la surface adhésive (10b) de la bobine (10b).

9. Kit de champs stériles comprenant une champ operatoire stérile selon la revendication 2, ou une tissu de recouvrement séparé (12c), dans lequel la surface adhésive de la bobine (10b) et toute surface adhésive du patient (14) sont recouvertes d'un film non adhésif, et la tissu (1) et la tissu de recouvrement (12c) sont chacune emballées individuellement de manière stérile.

10. Production d'un champ stérile ou d'un kit de champ stérile selon l'une des revendications précédentes, en vue d'un examen d'imagerie par résonance magnétique ou d'une intervention chirurgicale sur un patient, **caractérisée par** les étapes suivantes :
a. fourniture d'un tissu stérile flexible (1) et d'un revêtement plat (12b, 12c) ou d'un revêtement rigide (21) pour la couverture ou l'enrobage étanche aux germes d'un filament local (2) ;
b. préparation du tissu (1) pour la connexion à une bobine local (2) et/ou au revêtement (12b, 12c) ou au revêtement (21) par application d'une surface adhésive (10b), recouverte notamment d'un film non adhésif ;
c. fourniture d'une bobine local (2) ;
d. connexion du revêtement (21) ou de la bobine local (2) et/ou du revêtement (12b, 12c) au tissu (1).

11. Production selon la revendication précédente, dans laquelle les étapes c et d sont séparées spatialement et/ou temporellement des étapes précédentes, en particulier les étapes précédentes étant réalisées en usine, tandis que les étapes c et d sont réalisées uniquement sur le lieu d'utilisation du champ stérile.

12. Production selon la revendication précédente, dans laquelle la surface adhésive est recouverte à l'étape b d'un film non adhésif, qui est retiré avant ou au début de l'étape d, de plus, à l'étape a, un tissu de recouvrement séparé (12c) est prévu comme enveloppe de bobine (12b, 12c), et à l'étape b, le tissu (1) et le tissu de recouvrement séparé (12c) sont emballés individuellement de manière stérile, de sorte qu'après l'étape b, un kit de champ stérile selon la revendication 9 est disponible.

13. Utilisation d'un champ stérile selon l'une quelconque des revendications 1 à 8 ou d'un kit de champ stérile selon la revendication 9, comprenant les sous-étapes réalisées par une première personne stérile et une seconde personne, non nécessairement stérile, non nécessairement dans l'ordre suivant:
i. ouverture de l'emballage du champ stérile par la seconde personne, sans que celle-ci ne touche le champ stérile (100) lui-même ;
ii. retrait et dépliage du champ stérile (100) et retrait du film protecteur par la première personne ;
iii. dépose du champ stérile (100) déplié ;
iv. mise en contact de la bobine locale (2), non nécessairement stérile, avec la surface adhésive (10b) de la bobine par la deuxième personne, notamment de manière à positionner la bobine locale (2) sur une zone de bobine non adhésive ou peu adhésive (13b),
v. collage du revêtement sur le champ stérile (100) par la première personne en la pressant complètement sur la zone adhésive (10b), de manière à enfermer complètement la bobine locale (2) de manière stérile.

14. Utilisation selon la revendication précédente, utilisant un kit de champ stérile selon la revendication 9, dans laquelle :
- à la sous-étape ii, le film non adhésif est également retiré de la surface adhésive du patient (14) ; et à la sous-étape iii, le champ stérile déplié est placé sur le patient, la surface adhésive de la bobine tournée vers le haut, de sorte que la surface adhésive de la bobine (10b), et en particulier une ouverture qu'elle contient, se trouvent sur le site à examiner ou à opérer ; et à la sous-étape v, la bobine (2) est recouverte du tissu de recouvrement (12c) ;
ou
- d'abord, le tissu de recouvrement (12c) est placé sur une surface stérile, le côté non en contact avec la bobine étant situé en bas ; ensuite, la bobine locale (2), en particulier avec son ouverture concentrique à une ouverture du tissu de recouvrement (12c), est placée dessus; puis, conformément aux sous-étapes iii et iv, le champ stérile est placé, avec la surface adhésive de la bobine au dessus la bobine locale et en particulier avec une ouverture dans le tissu (1) concentrique à l'ouverture de la bobine locale, sur le tissu de recouvrement (12c) et puis la sous-étape v est réalisée.
